# EUROPEAN PATENT APPLICATION

(11) **EP 1 316 604 A1**
(43) Date of publication of application: **04.06.2003**
(21) Application number: 02014489.5
(22) Date of filing: 29.06.2002
(51) Int. Cl.: C12N 9/10, C12N 15/54, C12N 15/62, C12N 15/63, C12N 5/10, A61K 38/45, A61K 48/00, G01N 33/50

(54) **Novel thymidylate synthase mutants**

(30) Priority: 03.12.2001 US 334557 P
(71) Applicant: Geurtsen, Werner, Prof. Dr., Bainbridge Island, WA 98110 (US)
(72) Inventor: Loeb, L., Prof. Dr., Bellevue, WA 98005 (US); Geurtsen, Werner, Prof. Dr., 30539 Hannover (DE)
(74) Representative: Läufer, Martina, Dr.

(57) **Abstract**

Novel thymidylate synthase (TS mutants) are disclosed differing from human wild type thymidylate synthase in single, double, or multiple mutations, which show enhanced resistance to TS-inhibiting drugs like 5-fluorouracil or 5-fluoro-2-deoxyuridinemonophosphate. All these mutants can be used for the protection of normal human cell populations against the toxic manifestation of analogs that inhibited TS.

## Description

The invention is related to novel human thymidylate synthase mutants (thymidylate synthase mutants, TS mutants) and DNA encoding these mutants as well as the use thereof, especially in a pharmaceutical composition.

### Background of the invention

Various chemotherapeutic drugs, e.g., 5-fluorouracil (5-FU) or 5-fluoro-2-deoxyuridine monophosphate (FdUMP) inhibit the enzyme thymidylate synthase and as a result interfere with the DNA-metabolism of tumor cells, such as squamous cell carcinomas in the gastrointestinal area or breast tumors. The inhibition of TS is not specific to tumors but also occurs in normal cells. Treatment with ts inhibiting drugs results in a thymineless death of the affected cells.

Drugs that inhibit TS have been used in cancer chemotherapy for approximately 40 years for treatment of carcinomas. Analogues of the TS cofactor CH₂H₄-folate, such as Raltitrexed, Thymitaq, or AG331, also inhibit TS activity and have been used extensively in cancer chemotherapy. The anti-tumor activity of these folate analogues has been established (Landis et al., Nucleic Acid Res. 27:3702-3711 (1999), Tong et al., J. Biol. Chem. 273: 11611 - 11618 (1998) and J. Biol. Chem. 273:31209 - 31214 (1998). 5-Fluorouracil (5-FU) is used extensively for the treatment of carcinoma of the breast and colon. According to figures of the American Cancer Society new cases of cancer of the colon and of the breast, respectively, in the United States in 1997 had been estimated to 130,000 and 180,000 with 55,000 and 66,000 deaths, respectively, due to these cancers (American Cancer Society, Cancer Facts and Figures 151; 1997).

5-FU is effective either as a single agent or in combination with other drugs in ablation of these tumors. Additionally, novel specific antifolate based thymidylate synthase inhibitors are under development and await FDA approval.

The toxicity to bone marrow by this compound limits their effective dose. This toxicity is manifested by anemia, leukopenia, thombocytopenia. Further, numerous patients suffer from a mucositis in the gastro-intestinal tract during application of TS inhibitors, specifically 5-FU. It is believed that the specific and potent inhibition of thymidylate synthase accounts for both the therapeutic and toxic effects of 5-FU in animal cells (Henderson and Peterson, Nucleotide Metabolism, 1973 p. 226). Thymidylate synthase is a 72 kD protein that catalyses the methylation of dUMP to dTMP. In eukaryotic cells, DNA synthesis is dependent on the production of dTMP by this de *novo* pathway (Kornberg and Baker, DNA replication, 1992). In addition, it is known that TS activity is greatest in rapidly proliferating cells (Rode et al., JBC 225:1305, 1980). For these reasons, TS has been an important target for the design of chemotherapeutic agents.

The WO 98/33518 discloses thymidylate synthase mutants, which are resistant to chemotherapeutic effective folate analogues. The amino acid mutations are located at position 49, 52, 108, 221 or 225 of human wildtype TS. The resistance is explained with key positions of the respective mutations within the TS molecule. The theoretical background is thoroughly explained.

Thus, it was believed that mutations for enhanced resistance to 5-FU, 5-FdUR and others must be located at key positions for 5-FU-TS-interaction.

Due to the importance of TS inhibitors in cancer therapy there is an urgent need for new resistant mutants which can be used to protect normal human cell populations against the toxic manifestation of analogs that inhibit TS.

The object of the invention is to generate novel thymidylate synthase enzymes that produce enhanced resistance to 5-fluorouracil (5-FU), 5-fluorouridine (5-FdUR), and related drugs, and to make them available for therapeutic use.

According to this invention there are now provided a series of mutant human thymidylate synthase enzymes which show high resistance to the drug 5-FU and related analogs.

These mutant proteins and respective gene constructs are useful in transforming human non-tumorigenic cells (mucosal cells, bone marrow cells, etc.) either *in vitro* or *in vivo* prior or during the treatment of a patient receiving chemotherapy with TS-inhibiting drugs due to cancer or other diseases. The presence of these transformed cells will reduce the clinically severe myelo-suppression or alteration in gastrointestinal cells observed during treatment with the aformentioned drugs, as well as allow augmentation of the dose of chemotherapeutics to be implemented, since severe side effects due to this chemotherapy are reduced or eliminated.

The following human thymidylate synthase enzymes with single, double or multiple mutations are part of this invention:

### Thymidylate synthase mutants, differing from human wildtype thymidylate synthase in single mutations at positions:

E23, T53, V84, K93, D110, D116, M190, P194, S206, M219, H250, D254, Y258 or K284, where further silent or non-functional mutations with respect to TS activity are not excluded.
Prefered single mutations of the thymidylate synthase mutants cited above are E23G, D48V, T51S, T53S, V84A, K93E, D110E, D116A, M190L, P194Q, S206G, M219V, H250L, D254E, D254A, D254N, Y258S, Y258F or K284N.

### Thymidylate synthase mutants differing from human wildtype thymidylate synthase in double or multiple mutations at positions:

55, 106, and 284;
5, 78, and 219;
45 and 254;
193 and 231;
6, 69, and 211;
5, 13, and 231;
103 and 204;
142 and 225;
17, 116, and 254;
117, 169, and 254;
120 and 278;
38 and 104;
8, 81, 131, and 230;
51, 82, 99, and 171;
167 and 192,
   where further silent or non-functional mutations with respect to TS activity are not excluded.

### Prefered double or multiple mutations are:

T55I, V106A, and K284I;
G5S, R78C, and M219I;
V45A and D254N;
P193S and A231G;
S6N, D69Q, and Q211L;
G5D, L13R,and A231T;
S103T and V204A;
F142S and F225I;
A17T, D116A, and D254E;
F117S, K169R, and D254E;
S120T and K278R;
Q38H and K104D;
L8Q, W81G, L131V, and Y230F;
T51S, K82Q, K99D, and N171S;
T167S and L192P,

Unexpectedly numerous mutants have been found where the position of the mutati-on(s) is(are) quite remote from the center of activity of TS, especially remote from the Arg50 loop. Mutations throughout the sequence have been shown to influence resistance to folate analogs without adversely affecting TS enzyme activity.

Best results were obtained with the following mutants: D254N-D254A (double), D254E (single), T53S (single), Y258F (single), T53S-Y258F (double), T51S-K82Q-K99E-N171S (quadruple), G5D-L31R-A231T (triple), D254A (single), D254N (single). The results are given in greater detail in Table I.

The invention also encompasses recombinant cDNAs encoding for the above given mutants as well as vectors comprising these DNA sequences and - optionally - other components.

The invention also encompasses fusion proteins comprising a transductor for the transduction of a protein into a human cell and a human thymidylate synthase mutant of the invention coupled to said transductor. Methods for the production of fusion proteins can be taken from "Schwarze, et al., Science 285:1569, 1999". Transduction can also be achieved with the help of viral vectors, liposomes or complexing agents. The fusion proteins where an amino acid sequence attached to the TS mutant is used for protein transduction are well suited vor the introduction of the mutants of the invention into mucosal cells, especially oral mucosal cells, in culture or *in vivo.*

The TS mutants according to this invention can be expressed in either bacteria or yeast as host cells.

The protein mutants, respective DNAs, vectors or fusion proteins according to this invention can be used for transfection of human cells which may be affected by side effects due to chemotherapy with thymidylate synthase inhibitors, for the *ex-vivo* or *in vivo* transfection of bone marrow cells, or for transfection of early progenitor cells separated or grown from bone marrow cells. Vecotrs and DNAs can be used in gene therapy, where they are introduced into individuals harboring tumors. This is especially intended for protection of human mucosa against ulceration in patients undergoing chemotherapy with agents that inhibit TS. The means of this invention are also useful for introduction of TS mutants into normal (oral or other) mucosa in patients prior to chemotherapy.

The protein mutants of this invention, the respective DNAs, vectors or fusion proteins can also be used for the identification of new nucleotide analogs that inhibit normal human thymidylate synthase.

The protein mutants, respective DNAs, vectors or fusion proteins according to this invention are useful for the manufacture of pharmaceutical and gene therapeutic compositions for the protection of human mucosa against ulceration under chemotherapy with TS inhibitors. The invention encompasses such pharmaceutical or gene therapeutical compositions.

The inventors used errorprone PCR to mutagenize the fulllength human TS cDNA, and then selected mutants resistant to 5-fluorodeoxyuridine (5FdUR) in a bacterial complementation system. They found that resistant mutants contained one to five amino acid substitutions, and that these substitutions were located along the entire length of the polypeptide. Mutations were frequent near the active site Cys195 and in the catalytically important Arg50 loop; however, many mutations were also distributed throughout the remainder of the cDNA. Mutants containing a single amino acid replacement identified the following 14 residues as novel sites of resistance: Glu23, Asp48, Thr51, Thr53, Val84, Lys93, Asp110, Asp116, Met 190, Pro194, Ser206, Met219, His250, Asp254, Tyr258, and Lys284. Many of these residues are distant from the active site and/or have no documented function in catalysis or resistance. It was concluded that mutations distributed throughout the linear sequence and 3dimensional structure of human TS can confer resistance to 5FdUR. The findings imply that longrange interactions within proteins affect catalysis at the active site and that mutations at a distance can yield variant proteins with desired properties.

Amongst the TS variants containing a single amino acid substitution, the inventors identified replacements at residues that have not been previously reported to be sites of resistance in human TS. In fact, this more than doubles the number of single amino acid substitutions in human TS that have been demonstrated to yield 5FdUR resistance.

### EXPERIMENTAL PROCEDURES

### Cell lines and Materials

E. coli NM522 cells (Stratagene, La Jolla, CA) were used for cloning and library construction. E. coli c2913recA cells lacking TS (ΔthyA572, recA56), kindly provided by Dr. Daniel Santi (UCSF), were used in all complementation studies. Plasmid DNA was isolated by using Perfectprep Plasmid Mini (Eppendorf Scientific Inc., Westbury, NY) and Maxiprep kits (Qiagen, Charsworth, CA). Taq DNA polymerase and dNTPs were from Promega (Madison, WI). DNA oligomers and T4 DNA ligase were from Gibco Life Science Technology. Restriction enzymes were obtained from New England Biolab (Beverly, MA). DNA fragments were isolated by using the Quiaquick Gel Extraction Kit from Qiagen. 5-FdUR, FdUMP, thymidine were purchased from Sigma (St. Louis, MO), and (6R,S)-CH₂H₄-folate was obtained as the racemic mixture from Schircks Labs (Jona, Switzerland). ABI Prism Dye Terminator Cycle Sequencing kits for fluorescent sequencing were the products of Perkin Elmer (Branchburg, NJ).

### Standard and Error-prone PCR -

Standard PCR reactions contained 100 ng plasmid DNA carrying the wild type TS cDNA, 1 µM of the primers HK-MUT5' (5'ATAACAATTTCACACAGGAAACAGCTATGACC3) and HK-MUT3' (5'CAGGGTTTTCCCAGTCACGACGTTGTAAAACG3), 250 µM each dCTP, dTTP, dATP and dGTP, and 2 units of Taq DNA polymerase in 10 mM Tris-HCI (pH 8.3),1.5 mM MgCl₂, 50 mM KCI, 0.01% gelatin, 0.01% Triton X-100 in a total volume of 100 µl. Error-prone PCR was performed by the method of Cadwell and Joyce (17) with modification. Conditions were those described for standard PCR, except for elevation of the MgCl₂ concentration to 7 mM, addition of 0.5 mM MnCl₂ and unequal concentrations of the four dNTPs (1 mM dCTP, 1 mM dTTP, 0.2mM dATP ,0.1mM dGTP). PCR was performed in a PCT-100 Programmable Thermal Controller (MJ Research Inc., Watertown, MA) by using the following protocol: 3 min of initial denaturation at 94°C followed by 45 cycles of 94 °C for 1 min, 50 °C for 1 min and 72 °C for 3 min. To determine the mutation frequency under standard and mutagenic conditions, PCR products were cloned into the pCRII-TOPO vector (Invitrogen, Carlsbad, CA) and sequenced by using the primer, DLPCR-TS3R (5'-AAAAAAAAACCATGTCTCCGGATCTCTGGTAC-3').

### Construction of the TS mutant library

DNA amplified by error-prone PCR was digested with Sacl and Ndel and subjected to electrophoreses in an agarose gel. The 1 kb TS fragment was extracted from the gel by using the Qiaquick Gel Extraction kit (Qiagen) and ligated into the Ndel and Sacl-digested pGCHTS-TAA vector backbone (kindly provided from Dr. Daniel Santi) containing 115 bp of 5'-untranslated region from the L. casei TS gene as a leader sequence. The ligated plasmid DNA was transformed into NM522 E. coli cells (Stratagene) by electroporation (Gene Pulser, BioRad, 1.8 kV, 25 µF, 400 ohms).Twenty-five transformations were pooled and the size of the library was determined to be 2.8 x10⁶ independent clones by plating an aliquot of the transformation mixture on 2xYT medium containing 50 µg/ml of carbenicillin (Island Scientific, Bainbridge Island, WA). To determine the frequency and types of mutations introduced by PCR, plasmid DNA was isolated from the E. coli mutant library and sequenced by using the ABI Prism Dye Terminator Cycle Sequencing Kit and the primer DLPCR-TS3R. For amplification of the library, 25 ml of the pooled transformation mixture was added into 500 ml of 1xYT medium containing 50 µg/ml of carbenicillin, incubated overnight at 37 °C, and plasmid DNA was prepared. The plasmid DNA library (500 ng) was transformed into 100 µl of electrocompetent ₓ2913 cells. Transformation mixtures were pooled, grown overnight in appropriate antibiotics and stored at -80 °C in 15% glycerol.

### Genetic selection in E. coli

E. coli ₓ2913 cells harboring plasmids with random substitutions encoding different amino acids, the TS library, were grown overnight at 37 °C in 2xYT medium containing 50 mg/ml of carbenicillin and 50 mg/ml thymidine. Cultures were diluted 1:100 in the same medium and grown at 37 °C until the OD₆₀₀ reached 0.6 to 0.8. Aliquots (1 ml) of the exponentially growing cells were pelleted and resuspended in M9 salts and plated on three different media. On 1xYT containing 50 mg/ml of carbenicillin and supplemented with 50 mg/ml of thymidine, essentially all cells harboring the plasmid DNA should be recovered. On M9 plates containing carbenicillin, only cells expressing active TS proteins should grow because the medium lacks thymidine. Drugresistant clones were selected on M9 plates containing carbenicillin and increasing concentrations of 5FdUR essentially as previously described (Landis, D.M., Loeb, L.A., 1998, J. Biol. Chem. 273:25809-25817)

### Site-directed mutagenesis

Single amino acid substitutions were introduced by using the QuikChange site-directed mutagenesis kit (Stratagene). Plasmid DNA encoding the wild type TS cDNA was amplified by using PfuTurbo DNA polymerase and a set of primers containing the appropriate base ssubstitution. The PCR product was treated with Dpnl to cleave the parental DNA template and the noncleaved DNA was transformed into XL-1 Blue competent cells. Cells containing plasmid DNA with the desired substitution were selected on the LBampicillin agar plates, and plasmid DNA was harvested and sequenced.

### Purification of TS proteins

To subclone mutant TS fragments into the pHis vector (a modified pUC12 vector provided by A. Hizi), the TS cDNA was PCR-amplified by using HK-MUT5' and TS-Xho3' (5'- CAGCTCGAGCTCCTTTGAAAGCACCCTAAAC -3'). After digestion with *Ndel* and *Xhol,* the amplified fragments were ligated into the *NdelSall* digested pHis vector. The reconstructed plasmids were verified by both restriction analysis and DNA sequencing. Wild type and mutant TS proteins, as N-terminal hexahistidine fusions, were then purified by metal chelation chromatography on Ni²⁺ affinity resin (His Bind resin and buffer kit, Novagen), as previously described, with minor modifications (see Landis, cited above). An overnight culture of ₓ2913 cells expressing the His-TS fusion protein was diluted 1:100 into 250 ml of 2xYT medium containing carbenicillin and thymidine. After attaining an OD₆₀₀ of 0.2, cells were induced with 1 mM IPTG, grown to an OD₆₀₀ of 0.8, harvested by centrifugation, resuspended in 12 ml of 1X binding buffer (5 µM imidazole, 0.5 mM NaCI, 20 mM Tris-HCI, pH 7.9) and lysozyme (200 µg/ml, Sigma), and frozen at -80 °C. Frozen cells were thawed and lysed on ice for 3 hr. The disrupted cells were centrifuged (27,000 x g) and the supernatant was collected and applied to a charged 2.5 ml HisBind Column (1x2.5 cm, Novagen, Madison, WI). The resin was prepared by successive washes with 15 ml of deionized water, 10 ml of 1X charge buffer (50 mM NiSO₄), and 15 ml of 1X binding buffer. Following application of the crude extract, the column was washed with 30 ml of 1x binding buffer and 25 ml of a mixture containing 60% binding buffer and 40% wash buffer (60 mM imidazole, 500 mM NaCI, 20 mM Tris-HCI, pH 7.9). His-TS protein was eluted with 5 x 1 ml aliquots of 1X elution buffer (1 M imidazole, 0.5 mM NaCI, 20 mM Tris-HCI, pH 7.9). SDS-PAGE showed that TS protein was eluted in the second and third fractions. Fractions containing TS protein were combined and dialyzed against 50 mM Tris-HCI, pH 7.5, 1 mM EDTA, 200 mM NaCI and 10% glycerol for 16 h and then against the same buffer containing 1 mM dithiothreitol for additional 10 h. The concentration of purified TS was determined by using the Bradford assay.

### Kinetic analysis

TS activity was monitored spectrophotometrically by the increase in absorbance at 340 nm that occurs concomitant with the production of H₂folate (Δε=6400 M⁻¹ cm⁻¹). The reaction buffer contained 50 mM TES (pH 7.4), 25 mM NaCI, 6.5 mM formaldehyde, 1 mM EDTA and 150 µM 2-mercaptoethanol. When the concentration of dUMP was varied, a high concentration of (6R)-CH₂H₄folate was added (3001200 mM of a racemic mixture); when the concentration of (6R)-CH2H4-folate was varied, the concentration of dUMP was 400 µM. Purified wild type or mutant TS protein was added to initiate the reaction at 25 °C. Initial velocity measurements were obtained with a Perkin Elmer Lambda Bio 20 UV/VIS spectrophotometer. Steady-state kinetic parameters were derived by a nonlinear least squares fit of the data to the Michaelis-Menten equation with KaleidaGraph 3.0 software (Abelbeck Software, Reading PA). Inhibition constants (Ki) for 5-FdUMP were obtained from a Dixon plot by measuring initial velocities in the presence of dUMP, CH₂H₄-folate and inhibitor at 25 °C. Two dUMP concentrations (30 µM and 300 µM) were used, and FdUMP concentrations were varied. To initiate reactions, CH₂H₄-folate was added to yield a final concentration of 300 µM of the active isomer.

### RESULTS

Reference is taken to the attached figures.

### Figure legends

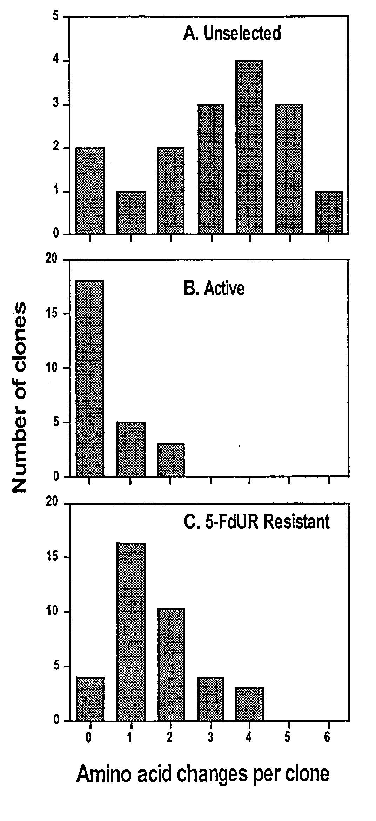

Fig. 1. Number of amino acid substitutions. A. Non-selected mutants; B. active mutants; C. 5-FdUR-resistant mutants. Unselected mutants were obtained from transformed bacterium grown in rich medium. Active mutants were obtained from bacteria grown on M9 minimal medium. 5-FdUR-resistant mutants were collected from cells grown on M9 medium containing 5-FdUR (200 nM or 300 nM). To confirm whether the plasmid DNA recovered from E. coli cells leads to the resistance to 5-FdUR, the recovered plasmid DNA was retransformed into fresh _2913 cells and the survival experiments were performed using varying amounts of 5-FdUR. Forty-three clones showing resistance to 5-FdUR in the second survival experiment were selected. The 630 bases, constituting two-thirds of the TS gene, were sequenced. Since the full length of TS cDNA is 939 bases long, 1.5 times as many substitutions are expected to be present throughout the protein.

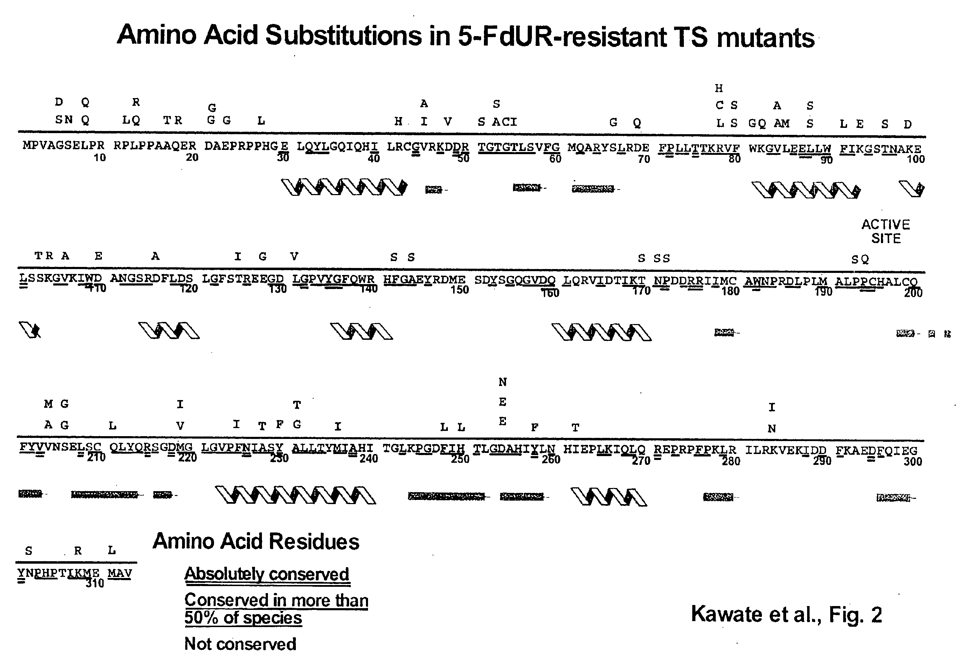

Fig. 2. Distribution of amino acid substitutions in 5-FdUR-resistant TS mutants. All of the amino acid substitutions detected in forty-one 5-FdUR-resistant mutants were presented. The amino acid residues of wild type TS protein were shown under the line. A double underlining indicates residues absolutely conserved among all TS proteins reported (1). A single underlining indicates residues that are conserved in more than 50 % of the TS proteins. Non-conserved residues are not underlined. The percent conservation was conservation was calculated by comparing thymidine synthases spanning viruses to mammals (1). You may want to designate single mutations by a color or box. Spiral shapes designate α-helices and arrows are β-sheets.

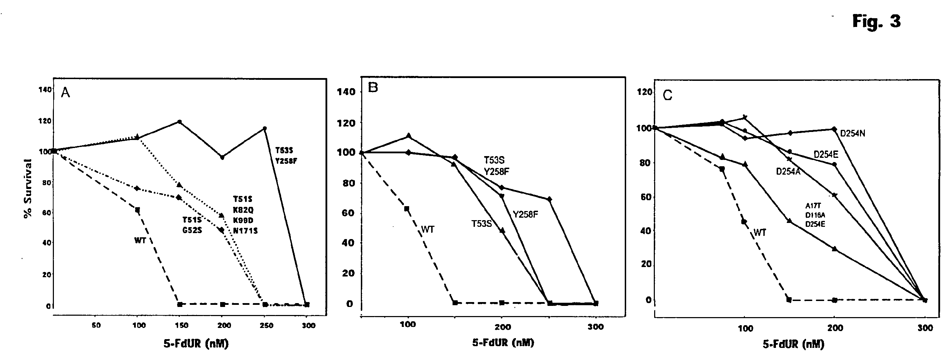

Fig. 3. Survival of 5-FdUR-resistant TS mutants. E. coli 2913 cells expressing wild type or mutant TS protein were grown on M9 minimal plates containing 5-FdUR for 48 h. Survival was determined by counting colonies at each dose of 5-FdUR and is expressed as a fraction of the survival of untreated cells. A. 5-FdUR-resistant mutants isolated in the present and previous experiments. Mutant 302, the most resistant clone in this library, carries two amino acids substitutions, T53S and Y258F. Survival is compared to that of the quadruple mutant T51S;K82Q;K99D;N171S (Mutant 362), the double mutant T51S; G52S, the most resistant in previous experiments (6); and the wild type. (B) Generation of single mutants. Mutant 302, carries two amino acid substitutions, T53S and Y258F. Each single mutant was made and the survival curve was compared to wild type and the parent mutant. (T53S was less resistant than Y258F. In this figure, the curve for T53S is that for T51S. The result was shown on p141 (02/08/2001). C. Mutants carrying amino acid substitutions at Asp254 residue. Four amino acid substitutions were accumulated at Asp254 residue (three of four substitutions were Asp to Glu changes and the other was an Asp to Asn substitution) Three different single mutants at Asp254 residue were created (D254E, D254N and D254A). Survivial curves of these single mutants and mutant 318, carrying three amino acid substitutions including D254E, were compared.

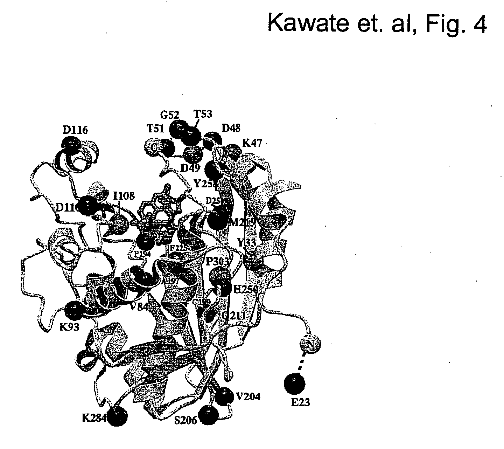

Fig 4. Location of amino acid substitutions in single 5-FdUR-resistant mutants in the threedimensional structure of human TS. A ribbon drawing of one subunit from the 1.9 Å structure of 32 dimeric human TS complexed with dUMP and ralitrexed, an antifolate drug, is shown, with the dimer interface furthest from the viewer. Residues 125 are disordered and are not in the crystallographic model; N and C represent the amino and carboxyl termini of residues 26313, respectively. The essential, active site Cys195 is represented by a green ball at its alpha carbon position. The ligands are shown in purple. All of the published amino acid substitutions in single, 5-FdUR-resistant mutants are mapped. The red balls denote PCR-generated mutations reported here; the blue balls denote mutations generated in this study by site-directed mutagenesis and mutations previously reported from this laboratory; the yellow balls denote mutations described by others. The coordinate set for the ternary complex was obtained from Protein Data Bank; chain 1HVY:A is modelled. The drawing, generously provided by Dr. Elinor Adman, was made by using the programs Molscript and Raster 3D.

### Table legends:

### Legend to Table II.

The resistance of each of the 41 mutants to 5-FdUR is expressed as fold enhancement in survival relative to wild type determined in parallel assays. All assays were carried out in duplicate at varrying concentration of 5-FdUR and most comparison were repeated in two or more separate experiments. As previously reported (5), cells expressing wild type TS exhibited a diminished ability to form colonies on M9 plates containing more than 150 nM 5-FdUR. In most but not all experiments no wild type colonies were observed at concentrations of 150 nM 5-FdUR or greater. Only those experiments in which the survival of the wild type was lower than 1 % were included in this tabulation. The percent survival of the wild type at the indicated concentrations of 5-FdUR is as follows: (a) 0.4 % in 0.4 nM; (b) <0.1 % in a 150 nM; (c) 0.1 % at 150 nM; (d) 0 at 300 nM; (e) 0.5 % at 150 nM; (f) 0 at 200 nM; (g) 0.95 at 150 nM.

### Mutagenesis of human TS by error-prone PCR

To introduce amino acid substitutions throughout the TS protein, error-prone PCR was carried out by increasing the concentration of Mg²⁺, adding Mn²⁺, and using unequal concentrations of the four dNTPs. Manganese ions have been shown to increase misincorporation by a variety of DNA polymerases, and the error-rates of these enzymes are proportional, over a limited range, to the relative concentrations of the dNTP substrates. The mutations introduced by PCR under standard and mutagenic conditions were compared by sequencing the initial two-thirds of the TS cDNA (nucleotides 1-630 encoding amino acids 1-210.) Under standard conditions with 1.5 mM Mg²⁺ and equimolar dNTPs, Taq DNA polymerase introduced a relatively small number of mutations; the average for the 36 clones sequenced was 0.2 mutations per clone. Under mutagenic conditions, however, the average was much higher, i.e., 9.8 mutations per clone, and the great majority of cDNAs carried more than 7 mutations (Table I). Six of 7 mutations found under standard conditions were A:T to G:C transitions, frequently introduced by Taq DNA polymerase. In contrast, under mutagenic conditions, A:T to T:A transversions were most frequent, and a variety of other types of substitutions were detected, predicting a greater diversity of amino acid substitutions.

### Construction of TS mutant library and analysis of unselected clones

Following amplification by error-prone PCR, the TS cDNAs were cloned into a plasmid vector downstream of the L. casei TS untranslated sequence. The ligated products of the backbone and these variant PCR products constituted the TS mutant library, containing ca. 2.8 x10⁶ independent clones. Plasmid DNA was isolated from 16 colonies obtained from growth on non-selective rich medium containing thymidine, and the number and types of nucleotide substitutions were determined by sequencing the 5'-proximal 630 nucleotides of the cDNA. The number of mutations per cDNA in the non-selected mutants is shown in Fig. 1A. Unselected clones have an average of 4.3 nucleotide changes, all of those detected were base substitutions with the exception of 2 deletions. At the amino acid level, this corresponds to 3.2 substitutions within the segment sequenced, suggesting that, on average, the full-length proteins contained about 4.8 amino acid replacements. Amino acid substitutions in the unselected clones were distributed fairly uniformly throughout the sequenced region (data not shown).

### Selection and analysis of active TS mutants

Expression of catalytically active TS protein enables TS-deficient E. coli ₓ2913 host cells to form colonies on M9 minimal plates in the absence of added thymidine. On plating the TS mutant library, approximately half the number of colonies was formed on M9 minimal medium lacking thymidine relative to the thymidine-supplemented medium. Thus, approximately 50% of the TS mutants exhibited sufficient catalytic activity to complement the TS-deficient phenotype. Of the active mutants analyzed, 8 of 18 clones carried amino acid substitutions in the 630 bp sequenced (Fig. 1B). No amino acid substitutions were detected in the other 10 clones, although two clones contained silent mutations. This limited survey indicates that most of the active mutant clones contained less than one amino acid substitution and that these substitutions do not prevent the catalytic activity required for complementation.

### Isolation and analysis of 5-FdUR-resistant TS mutants

To isolate mutants that render E. coli ₓ2913 resistant to 5-FdUR, the TS library was plated on M9 minimal medium containing 200 or 300 nM 5-FdUR. As previously reported, cells expressing wild type TS exhibited a diminished ability to form colonies on M9 plates containing more than 150 nM 5-FdUR. Resistant colonies were selected and plasmid DNA was isolated and re-transformed into fresh ₓ2913 cells to confirm that the plasmid was the source of the drug-resistant phenotype. Thirty-six independent clones were picked that showed increased resistance upon re-exposure to 5-FdUR, and the 5'-terminal 630 bp of the cDNA was sequenced. Zero to 4 amino acid substitutions were observed in the sequenced segment of resistant clones, the average being 1.6 (Fig. 1C).

To obtain a profile of amino acid replacements conferring resistance, the remaining, 3'-proximal third of the cDNA from each of the 38 mutants was sequenced. In two of the clones we failed to detect any substitutions; the mechanisms of resistance in these two clones remains to be determined. A summary of all predicted amino acid substitutions found in 36 of the mutants that exhibited resistance to ≥150 nM 5-FdUR is recorded in Table II. Resistance is recorded as percent survival relative to percent survival of host cells harboring wild type TS at 150 nM 5-FdUR of greater. Expression of TS in 10 of the resistant mutants was analyzed by Western blotting with antiserum directed against human TS. The level of expression of wild type TS was more than 2-fold greater than that of any of the mutants, estimated by visual inspection of the gel (data not shown). The mutant proteins may be more labile than the wild type or more susceptible to proteolytic degradation.

### Distribution of PCR-generated mutations yielding 5-FdUR resistance

The distribution of amino acid substitutions in the 5-FdUR-resistant clones generated by error-prone PCR is indicated in Fig. 2. In the case of mutants with multiple substitutions, not all of the replacements are expected to be directly involved in resistance, some presumably having been co-selected. Importantly, substitutions were dispersed throughout the protein. The distribution included "hot spots" where clustered mutations appeared to be more frequent than would be expected on a random basis, as well as "cold spots." Several regions exhibited a high density of mutations. The residues between and including Val45 and Thr55 constituting the Arg50-loop harbored 7 different substitutions. The replacements included T51S, commonly observed, and present in most resistant mutants, in previous experiments. These results are in accord with previous reports showing that the evolutionarily conserved Arg50-loop is a major site of mutations that confer 5-FdUR resistance.

The residues from Arg78 to Leu88 accumulated 9 replacements. This region has not been reported to be involved in 5-FdUR resistance; structural analysis indicates that it is located on the active site pocket and interacts with folate-based inhibitors. The N-terminal residues from Gly5 through Gln18 accumulated 10 substitutions; this segment is disordered in existing crystal structures and has no known function. There is no obvious correlation between the frequency of either single or multiple substitutions observed in resistant mutants and the presence of beta sheets or alpha helices. However, amino acid residues that are completely conserved during evolution are less likely to incur substitutions resulting in 5-FdUR resistance.

### Resistance of T53S;Y258F reflects the contribution of both single mutations

Amongst the newly identified clones, the double mutant T53S;Y258F (clone 302), exhibited a high level of resistance at elevated concentrations of 5-FdUR. In multiple experiments, T53S;Y258F was as resistant or more resistant to elevated concentrations of 5-FdUR than any clone tested. As shown in Fig. 3A, E. coli cells carrying the T53S;Y258F mutant grew on minimal plates containing 250 nM 5-FdUR, whereas, in a side-by-side comparison, the double mutant T51S;G52S, formed colonies at 200 nM, but not 250 nM, 5-FdUR. The same was true for another clone that exhibited highlevel resistance, the quadruple mutant T51S;K82Q;K99E;N171S (clone 362), as well as for another triple mutant G5D;L31R;A231T (clone 315, results not shown). To analyze the contribution of individual amino acid substitutions to the 5-FdUR resistance of the double T53S;Y258F mutant, we generated the T53S and Y258F single mutants by site-directed mutagenesis. As illustrated in Fig. 3B, both the T53S and Y258F single mutants were less resistant than the double mutant, but more resistant than wild type TS. These results indicate that both substitutions contribute to the high level of resistance exhibited by T53S;Y258F.

### Asp254 is a site of 5-FdUR resistance

Replacements at Asp254 were detected in 3 multiply substituted, resistant clones generated by error-prone PCR (D254E in 2 clones, and D254N in one). We generated several single mutants at this site and assessed their resistance. As shown in Fig. 3C, the three mutants D254A, D254E and D254N all showed higher resistance than wild type TS and were more resistant than a parent clone (clone 318) which had two substitutions in addition to D254E (A17T;D116A; D254E ).

### Kinetic Analysis of Mutant TS Proteins

To establish that the resistance to 5-FdUR observed in E. coli reflects altered function of the mutant proteins, several TS variants were purified as N-terminal hexahistidine fusions. Purity was estimated to be greater than 80% by densitometric scanning of Coomassie Blue-stained gels. Steady-state kinetic parameters for the wild type and mutant TS proteins are recorded in Table III. The kcat values observed for the mutants, and the Km values for dUMP, do not differ markedly from that of wild type. However, the Km values of the mutants for CH₂H₄-folate were 5- to 13-fold higher than that for wild type TS. Km's were approximately 10-fold higher for the double mutant T53S; Y258F (clone 302) and the quadruple mutant T51S; K82Q; K99D; N171S (clone 362), which were among the most resistant variants. In accord with the E. coli survival data, the Ki of all mutants for 5-FdUMP was greater than that of wild type TS. Mutant T53S; Y258F (clone 302), which exhibited high resistance to 5-FdUR in our complementation assay, showed a 6-fold increase. T51S demonstrated the largest increase over wild type (11-fold), while the less resistant single mutant, Y258F (see Fig. 3B), showed a lesser increase (3.1 fold).

### Single mutants that yield 5-FdUR resistance

The hypothesis we set out to assess is that mutations in TS that confer 5-FdUR resistance are distributed throughout the protein, and are not confined to regions directly involved in catalysis. In this work, we created single mutants that newly identify a total of 14 amino acids as residues where 5-FdUR resistance can arise. Eight residues were identified in mutants created by error-prone PCR; these are E23, V84, K93, D110, P194, S206, H250, K284. Six of these (E23, V84, K93, D110, S206, K284) are located at a distance from the active site and are not associated with catalysis. We also identified other single amino acid substitutions that may confer 5-FdUR resistance among the PCR variants containing multiple mutations. Four of these (T51, T53, D254 ,Y258) were chosen, and verified to be authentic sites of resistance by using site-directed mutagenesis to create single mutants; these amino acids reside within the active site and have been implicated in catalysis (1). An additional two residues (D116 and M219) were also shown by site-directed mutagenesis to yield resistance mutations (D116A and M219V, respectively.) The location in the three dimensional structure of all the single mutations analyzed here, together with the location of other previously identified resistance mutations, is shown in Fig. 4.

### DISCUSSION

In the course of investigations it was found that the resistant variants, listed in Table II, harbor substitutions throughout the linear sequence (Fig. 2) and threedimensional structure (Fig. 4) of the protein. Some of the 74 different replacements (or combinations thereof) are responsible for 5-FdUR resistance, including the substitutions in single mutants, while other replacements are presumably co-selected and not relevant to resistance.

Recent crystallograhic analyses of TS, especially closed ternary complexes with dUMP and folate-based inhibitors, allowed to evaluate these replacements with a view toward understanding possible structure function relationships, and establishing fresh targets for directed mutagenesis. We have used the atomic coordinates of the wild type amino acids in tightly closed complexes with dUMP and a folate analog inhibitor [Protein Data Bank (NCBI, National Center for Biotechnology Information, U.S.A., GenBank® entries) 1HVY and 1JU6, respectively] to locate each amino acid replacement in the threedimensional structure, and to consider possible bases for resistance.

### Single mutants

The single mutants generated by PCR (see Table II) contain amino acid replacements that are distributed throughout the protein. Possible resistance mechanisms can be envisioned for some of the replacements, while no mechanisms are apparent for others. For example, substitution of valine for glutamate in the D48V mutant would disrupt the hydrogen bonding network within the Arg50-loop that mediates dUMP binding, and also affect interactions with the second subunit of the obligate homodimer; it has been proposed that mutations at residues 4752 may confer resistance by destabilizing the closed ternary complex. At position 84 in the V84A mutant, wild type valine interacts with two residues, Phe80 and Phe225, that contact the cofactor. Altered interactions with folate are known to confer 5-FdUR resistance, and could account for the resistance in the mutant. At position 110, wild type aspartate interacts with Trp109, which in turn interacts with both dUMP and cofactor, perhaps accounting for resistance of the D110E mutant. At position 194 in the active site, wild type proline interacts with the catalytic cysteine, presumably accounting for the resistance of the P194L mutant. At position 250, wild type histidine interacts with several residues that contact dUMP, including Asp226, presumptively accounting for the resistance of the H250L mutant. In contrast, resistance mechanisms for the single mutants E23G, K93E, S206G and K284N are not apparent. As shown in Fig. 4., E23G is in the disordered N-terminal region, while K93E, S206G and K284 are located in loops at the periphery of the TS monomer, and have no obvious relationship to catalysis or resistance. By using site-directed mutagenesis to create single mutants, we showed that several amino acids which were replaced in multiply mutated PCR variants are authentic sites of 5-FdUR. Among these site-directed replacements is T51S, the most common mutation previously observed and present in the highly resistant variant T51S, G52S. Thr51 participates in H-bonding within the Arg50-loop, and with Val313 after ligand-induced conformational changes of the C-terminal segment (1).
5-FdUR resistance could arise at Thr51 from several mechanisms, including an effect on this conformational change. Two replacements at Asp254 (D254N and D254E) were observed in multiply mutated variants (255, 318, 358), and we found that both, as well as the D254A substitution, conferred resistance as single mutations (Fig. 203C). Asp254 is H-bonded to His256, which is in turn H-bonded to dUMPbinding Arg175 in the second subunit; 5-FdUR resistance may arise from disruption of these interactions. We also observed that each replacement in the double mutant Y258S, T53S conferred resistance as a single mutation (Fig. 3B). Whereas the resistance of T53S may be attributable to disturbance of H-bonding in the Arg50-loop, the resistance of Y258F is likely due to loss of H-bonding between the tyrosine hydroxyl group and dUMP and/or Arg175'. The D116A substitution, like some of the PCR-generated single replacements, lies at the surface of the protein, and its contacts do not suggest why it confers resistance. Lastly, the D219V replacement affects Met219, which has multiple contacts with Tyr33, a known site of resistance, and with His256, which interacts with dUMP. Replacements in multiply mutated variants - The resistance arising from multiple substitutions is difficult to analyze. Nonetheless, evaluation of the replacements in the double and higher mutants listed in Table II and Fig. 2 can afford potentially useful inferences and point to particular amino acids for further examination. It is a logical assumption that regions where mutations cluster may have special relevance for resistance. One such region includes residues Val45 through Thr55 constituting the Arg50-loop. Wild type residues at the 6 mutated positions (45,48,51, 53, 55) participate in a network of interactions, primarily H-bonding, within the loop, and also between theArg 50-loop and the second subunit that contains dUMP-binding residues 175' and 176'. Replacements at these 6 positions might affect interactions with both dUMP and 5FdUMP, and also destabilize the ternary complex, thereby conferring resistance. A conspicuous cluster of mutations was noted at positions Arg78 through Leu88, where 7 residues harbor 9 different replacements. Phe 80 in this region interacts with the co-factor, while Glu87 may have destabilizing interactions with both the co-factor and dUMP. Wild type amino acids at four mutated positions (78, 81,82,84) contact Phe80, while those at three mutated positions (84,85,88) interact with Glu87. Altered interactions with folate and/or dUMP may account for the resistance conferred by replacements in this region. Of particular interest with respect to folate binding is the occurrence of the M311L replacement in the double mutant 217. Met311, together with three wild type residues that yield 5-FdUR resistant mutants, form a hydrophobic collar around the paminobenzoic acid ring in the folate analog in a new ternary complex. A third group of 14 replacements was observed in the N-terminal 27 amino acids, which are disordered in crystal structures (1). This observation raises the possibility that at least some wild type amino acids in the N-terminal region may have an unrecognized function(s) in 5-FdUR resistance, a possibility substantiated by identification of the single mutant E23G. A related possibility is that the N-terminus may have a specific function(s) and an ordered structure(s) in conformational states which have not been captured in existing crystals.Another possible source of 5-FdUR resistance in multiply mutated variants is altered interactions between the subunits of the obligate TS homodimer; these interactions may be important for positioning of dUMP-binding residues in one or both subunits, and may thus affect 5-FdUR resistance. For example, Val45 in the Arg50-loop interacts with V204 in the second subunit, and we have previously isolated a 5-FdUR-resistant V204D mutant. The Val204Val45 hydrophobic interaction was disrupted in four different multiply substituted mutants isolated here, two involving Val45 (mutants 255 and 326) and two involving Val 204 (mutants 316 and 359). In another example of subunit interactions, Glu211 resides at the dimer interface where it contacts Phe59 from the second subunit. We have previously identified the 5-FdUR resistance mutation Q211L in a single mutant, and isolated it again here in the triple mutant 313. All told, we can offer a plausible structure-based suggestion for the resistance of most of the 35 mutants in Table II, though only 5 of 29 contain a single substitution. These mutants contain a replacement at a position where the wild type residue is either: (a) In direct contact with dUMP or a residue that contacts dUMP; and/or (b) in direct contact with folate or a residue that contacts folate; and/or (c) interacts with the second subunit at the dimer interface. We and others have previously documented 5-FdUR resistance mutations in these three categories. We rationalize our observations concerning resistance mutations at ligand-interacting residues as follows.

Wild type amino acids that interact directly with substrates or co-factors are likely to be important for catalytic activity, and to yield relatively few replacements that conserve catalytic efficiency, alter substrate/inhibitor preference and afford resistance. This relative lack of mutability not with standing, drug resistance mutations at such residues could be identified by intensive, targeted mutagenesis. On the other hand, residues that contact ligand-interacting residues may yield a relatively large number of substitutions that simultaneously preserve activity, modulate substrate/inhibitor interactions and confer drug resistance; mutations at such residues may be highly prevalent in the present work, where each residue in the protein has a limited probability of being substituted. We can also suggest possible sources of 5-FdUR resistance for the four single amino acid replacements that are far removed from the active site in current crystal structures. We have discussed the E23G mutation in the disordered, N-terminal segment, and noted that three others (K93E, S206G and K284) are located in loops at the surface of the monomer (Fig. 4). If TS functions in a multiprotein complex in vivo, these loops may be involved in proteinprotein interactions that after the conformation of TS and affect resistance indirectly. It is also possible that the loops are involved in the conformational dynamics of the protein, and affect motions that influence the probability of reactions at the active site. Studies of single enzyme molecules by confocal fluorescence spectroscopy indicate that enzymes undergo sets of transient oscillations during each catalytic step. Amino acid substitutions at a distance may affect these oscillations, and thereby alter substrate and inhibitor discrimination at the active site . Perhaps yettobe determined mechanisms that govern conformational changes during catalysis can be affected by amino acid replacements throughout the protein.

### REFERENCES

1. Carreras, C. W., and Santi, D. V. (1995) Annu. Rev. Biochem. 64, 721762
2. Ahmad, S. I., Kirk, S. H., and Eisenstark, A. (1998) Annu. Rev. Microbiol, 52, 591625
3. Rustum, Y. M., Harstrick, A., Cao, S., Vanhoefer, U., Yin, M. B., Wilke, H., and Seeber, S. (1997) J. Clin. Oncol. 15, 389400
4. Danenberg, P. V. , Malli, H., and Swenson, S. (1999) Semin. Oncol. 26, 621631

Protein Data Bank NCBI, National Center for Biotechnology Information, U.S.A. (GenBank®); world wide web: www.ncbi.nlm.nih.gov: Einträge: 1 JUJA. Chain, A., Human Thymidylate Synthase; P04818 TYSY_Human; XP_046765 Thymidylate synthase homo sapiens; BAB 83676 Thymidylate Synthase homo sapiens

### Abbreviations

The abbreviations used are: CH₂H₄-folate, (6R,S)-N⁵,N¹⁰-methylene-5,6,7,8-tetrahydrofolate; 5-FdUR, 5-fluoro-2'-deoxyuridine; FdUMP, 5-fluoro-2'-deoxyuridine 5'-monophosphate; 5-FU, 5-fluorouracil; bp, base pair(s); PCR, polymerase chain reaction; PAGE, polyacrylamide gel electrophoresis; TES, N-tris[hydroxymethyl]-methyl-2-aminoethane-sulfonic acid; TS, thymidylate synthase.

The human numbering system of TS is used for consistency. H256 corresponds to H207 in E. coli, Asp254 is Asp209 in E. coli, Arg175' is Arg126' in E. coli, and Tyr258 is Tyr 261 in E. coli. The prime indicates residues contributed by the opposing homodimer.

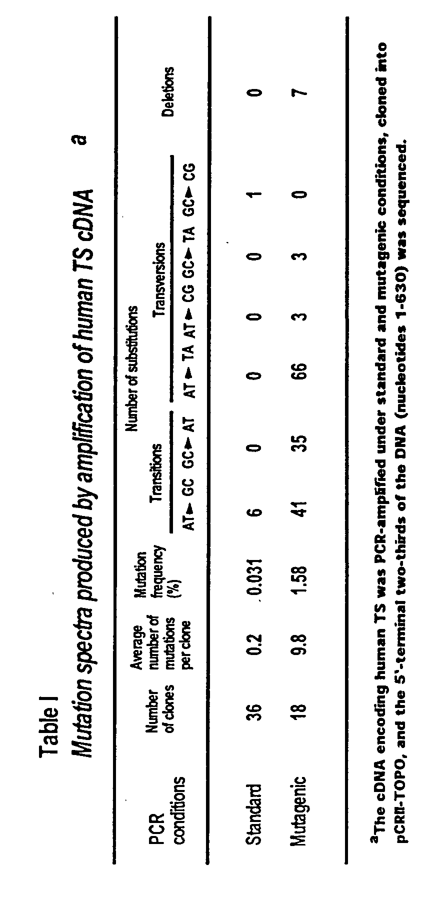

**Table II**

| *5-FdUR-resistant human TS mutants created by error-prone PCR*^{a,b} | | | |
|---|---|---|---|
| | Substitution(s) | Mutant | |
| | Survival^{c} | number | (Mutant/WT) |
| Wild type | none | --- | |
| Single mutants | E23G | 357 (a) | >110 |
| | D48V | 312 (a) | >98 |
| | V84A | 319 (b) | 54 |
| | K93E | 209 (c) | 340 |
| | D110E | 330 (b) | 530 |
| | P194Q | 226 (d) | 36 |
| | S206G | 229 (e) | >24 |
| | H250L | 339 (a) | >7 |
| | K284N | 331 (a) | >14 |
| Double mutants | P12L, N302S | 307 (f) | >37 |
| | D21G, M311L | 217 (d) | 56 |
| | V45A, D254N | 255 (e) | >210 |
| | T53S, Y258F | 302 (g) | 220 |
| | G54C, M23 | 329 (f) | >180 |
| | F80S, F91L | 219 (h) | >90 |
| | F80S, L88S | 353 (f) | >250 |
| | L85M, L88S | 361 (i) | >62 |
| | T96S, A228T | 370 (e) | >300 |
| | S103T, V204A | 316 (f) | >140 |
| | F142S, F225I | 317 (f) | >200 |
| | P172S, D254E | 358 (e) >180 | |
| | P193S, A231G | 308 (g) 270 | |
| Triple mutants | G5D, L13R, A231T | 315 (g) | 280 |
| | G5S, R78C, M2191 | 228 (e) | >82 |
| | S6N, D69Q, Q211L | 313 (f) | >140 |
| | A17T, D116A, D254E | 318 (b) | 770 |
| | Q18R, R78H, I262T | 238 (j) | 54 |
| | T55I, V106A, K284I | 222 (h) | >48 |
| | S66G, K104R, E128G | 369 (f) >210 | |
| Quadruple and higher | L8Q, D21G, T53A, R78L | 203 (e) | >320 |
| | L8Q, W81G, L131V, Y230F | 354 (f) >170 | |
| | L13Q, P27L, R42H, V84A, F248L | 356 (f) | >190 |
| | V45I, T125I, A144S, K308R | 326 (b) 16 | |
| | T51S, K82Q, K99D, N171S | 362 (g) 180 | |
| | T170S, V204M, S206G, M219V | 359 (f) >160 | |

☐1: 1JUJA. Chain A, Human
Th...[gi:15988342]
BLink, 3D Domains, Related Sequences, PubMed,
Structure, Taxonomy, LinkOut

LOCUS       1JUJ_A                  313 aa            linear   PRI 24-AUG-2001
DEFINITION  Chain A, Human Thymidylate Synthase Bound To Dump And Ly231514, A
            Pyrrolo(2,3-D)pyrimidine-Based Antifolate.
ACCESSION   1JUJ_A
PID         g15988342
VERSION     1JUJ_A  GI:15988342
DBSOURCE    pdb: molecule 1JUJ, chain 65, release Aug 24, 2001;
            deposition: Aug 24, 2001;
            class: Transferase;
            source: Mol_id: 1; Organism_scientific: Homo Sapiens;
            Organism_common: Human; Expression_system: Escherichia Coli;
            Expression_system_common: Bacteria; Expression_system_strain: Dh5a;
            Expression_system_vector_type: Plasmid; Expression_system_plasmid:
            Pgchts-Taa;
            Non X-ray method: X-Ray Diffraction.
KEYWORDS    .
SOURCE      human.
  ORGANISM  Homo sapiens
            Eukaryota; Metazoa; Chordata; Craniata; Vertebrata; Euteleostomi;
            Mammalia; Eutheria; Primates; Catarrhini; Hominidae; Homo.
REFERENCE   1  (residues 1 to 313)
  AUTHORS   Schiffer,C.A., Clifton,I.J., Davisson,V.J., Santi,D.V. and
            Stroud,R.M.
  TITLE     Crystal structure of human thymidylate synthase: a structural
            mechanism for guiding substrates into the active site
  JOURNAL   Biochemistry 34 (50), 16279-16287 (1995)
  MEDLINE   96110704
REFERENCE   2  (residues 1 to 313)
  AUTHORS   Sayre,P.H., Finer-Moore,J.S., Fritz,T.A., Biermann,D., Gates,S.B.,
            Mackellar,W.C., Patel,V.F. and Stroud,R.M.
  TITLE     Multi-Targeted Antifolates Aimed At Avoiding Drug Resistance Form
            Covalent Closed Inhibitory Complexes With Human And Escherichia
            Coli Thymidylate Synthase
  JOURNAL   Unpublished
REFERENCE   3  (residues 1 to 313)
  AUTHORS   Sayre,P.H., Finer-Moore,J.S., Fritz,T.A., Biermann,D., Gates,S.B.,
            Mackellar,W.C., Patel,V.F. and Stroud,R.M.
  TITLE     Direct Submission
  JOURNAL   Submitted (24-AUG-2001)
COMMENT     Revision History:
            SEP 19 1 Initial Entry.
FEATURES             Location/Qualifiers
     source          1..313
                     /organism="Homo sapiens"
                     /db_xref="taxon:9606"
     Region          1..273
                     /region_name="Domain 1"
                     /note="NCBI Domains"
     SecStr          31..42
                     /sec_str_type="helix"
                     /note="helix 1"
     SecStr          45..48
                     /sec_str_type="sheet"
                     /note="strand 1"

SecStr 54..60
/sec_str_type="sheet"
/note="strand 2"
SecStr 61..68
/sec_str_type="sheet"
/note="strand 3"
SecStr 81..93
/sec_str_type="helix"
/note="helix 2"
SecStr 115..122
/sec_str_type="helix"
/note="helix 3"
SecStr 134..141
/sec_str_type="helix"
/note="helix 4"
SecStr 160..170
/sec_str_type="helix"
/note="helix 5"
SecStr 177..181
/sec_str_type="sheet"
/note="strand 4"
SecStr 195..205
/sec_str_type="sheet"
/note="strand 5"
Het bond(195)
/heterogen="(UMP, 314 )"
SecStr 206..214
/sec_str_type="sheet"
/note="strand 6"
SecStr 215..219
/sec_str_type="sheet"
/note="strand 7"
SecStr 223..241
/sec_str_type="helix"
/note="helix 6"
SecStr 245..252
/sec_str_type="sheet"
/note="strand 8"
SecStr 253..259
/sec_str_type="sheet"
/note="strand 9"
SecStr 261..269
/sec_str_type="helix"
/note="helix 7"
SecStr 277..282
/sec_str_type="sheet"
/note="strand 10"
SecStr 295..300
/sec_str_type="sheet"
/note="strand 11"
ORIGIN
1 mpvagselpr rplppaaqer daeprpphge lqylgqiqhi lrcgvekddr tgtgtlsvfg
61 mqaryslrde fpllttkrvf wkgvleellw fikgstnake lsskgvkiwd angsrdflds
121 lgfstreegd lgpvygfqwr hfgaeyrdme sdysgqgvdq lqrvidtikt npddrriimc
181 awnprdlplm alppchalcq fyvvnselsc qlyqrsgdmg lgvpfniasy alltymiahi
241 tglkpgdfih tlgdahiyln hieplkiqlq reprpfpklr ilrkvekidd fkaedfqieg
301 ynphptikme mav
//

Revised: October 24, 2001.

Disclaimer | Write to the Help Desk
NCBI | NLM | NIH

1: P04818. Thymidylate synth...[gi:136611]    BLink, OMIM, Related Sequences, PubMed, Taxonomy, LinkOut

LOCUS       TYSY_HUMAN                313 aa            linear   PRI 01-MAR-2002
DEFINITION  Thymidylate synthase (TS) (TSase).
ACCESSION   P04818
PID         g136611
VERSION     P04818  GI:136611
DBSOURCE    swissprot: locus TYSY_HUMAN, accession P04818;
            class: standard.
            created: Aug 13, 1987.
            sequence updated: Mar 1, 1989.
            annotation updated: Mar 1, 2002.
            xrefs: gi: 37478, gi: 37479, gi: 220135, gi: 220136, gi: 12803482,
            gi: 12803483, gi: 15530266, gi: 15530267, gi: 220133, gi: 2160415,
            gi: 279460, pdb accession 1HVY, pdb accession 1HW3, pdb accession
            1HW4
            xrefs (non-sequence databases): MIM 188350, InterPro IPR000398,
            Pfam PF00303, PRINTS PR00108, ProDom PD001180, PROSITE PS00091
KEYWORDS    Transferase; Methyltransferase; Nucleotide biosynthesis;
            3D-structure.
SOURCE      human.
  ORGANISM  Homo sapiens
            Eukaryota; Metazoa; Chordata; Craniata; Vertebrata; Euteleostomi;
            Mammalia; Eutheria; Primates; Catarrhini; Hominidae; Homo.
REFERENCE   1  (residues 1 to 313)
  AUTHORS   Takeishi,K., Kaneda,S., Ayusawa,D., Shimizu,K., Gotoh,O. and
            Seno,T.
  TITLE     Nucleotide sequence of a functional cDNA for human thymidylate
            synthase
  JOURNAL   Nucleic Acids Res. 13 (6), 2035-2043 (1985)
  MEDLINE   85215597
  REMARK    SEQUENCE FROM N.A.
REFERENCE   2  (residues 1 to 313)
  AUTHORS   Kaneda,S., Nalbantoglu,J., Takeishi,K., Shimizu,K., Gotoh,O.,
            Seno,T. and Ayusawa,D.
  TITLE     Structural and functional analysis of the human thymidylate
            synthase gene
  JOURNAL   J. Biol. Chem. 265 (33), 20277-20284 (1990)
  MEDLINE   91056070
  REMARK    SEQUENCE FROM N.A.
REFERENCE   3  (residues 1 to 313)
  AUTHORS   Strausberg,R.
  TITLE     Direct Submission
  JOURNAL   Submitted (-SEP-2001)
  REMARK    SEQUENCE FROM N.A.
            TISSUE=Bone marrow, and Placenta
REFERENCE   4  (residues 1 to 313)
  AUTHORS   Takeishi,K., Kaneda,S., Ayusawa,D., Shimizu,K., Gotoh,O. and
            Seno,T.
  TITLE     Human thymidylate synthase gene: isolation of phage clones which
            cover a functionally active gene and structural analysis of the
            region upstream from the translation initiation codon
  JOURNAL   J. Biochem. 106 (4), 575-583 (1989)
  MEDLINE   90110051
  REMARK    SEQUENCE OF 1-67 FROM N.A.
REFERENCE   5  (residues 1 to 313)
  AUTHORS   Shimizu,K., Ayusawa,D., Takeishi,K. and Seno,T.

TITLE Purification and NH2-terminal amino acid sequence of human
thymidylate synthase in an overproducing transformant of mouse FM3A
cells
JOURNAL J. Biochem. 97 (3), 845-850 (1985)
MEDLINE 85261174
REMARK SEQUENCE OF 1-24.
REFERENCE 6 (residues 1 to 313)
AUTHORS Schiffer,C.A., Clifton,I.J., Davisson,V.J., Santi,D.V. and
Stroud,R.M.
TITLE Crystal structure of human thymidylate synthase: a structural
mechanism for guiding substrates into the active site
JOURNAL Biochemistry 34 (50), 16279-16287 (1995)
MEDLINE 96110704
REMARK X-RAY CRYSTALLOGRAPHY (3.0 ANGSTROMS).
REFERENCE 7 (residues 1 to 313)
AUTHORS Phan,J., Koli,S., Minor,W., Dunlap,R.B., Berger,S.H. and Lebioda,L.
TITLE Human thymidylate synthase is in the closed conformation when
complexed with dUMP and raltitrexed, an antifolate drug
JOURNAL Biochemistry 40 (7), 1897-1902 (2001)
MEDLINE 21229106
REMARK X-RAY CRYSTALLOGRAPHY (1.9 ANGSTROMS).
REFERENCE 8 (residues 1 to 313)
AUTHORS Phan,J., Steadman,D.J., Koli,S., Ding,W.C., Minor,W., Dunlap,R.B.,
Berger,S.H. and Lebioda,L.
TITLE Structure of human thymidylate synthase suggests advantages of
chemotherapy with noncompetitive inhibitors
JOURNAL J. Biol. Chem. 276 (17), 14170-14177 (2001)
MEDLINE 21216721
REMARK X-RAY CRYSTALLOGRAPHY (2.0 ANGSTROMS).
COMMENT -------------------------------------------------------------------
This SWISS-PROT entry is copyright. It is produced through a
collaboration between the Swiss Institute of Bioinformatics and
the EMBL outstation - the European Bioinformatics Institute.
The original entry is available from http://www.expasy.ch/sprot
and http://www.ebi.ac.uk/sprot
-------------------------------------------------------------------
[CATALYTIC ACTIVITY] 5,10-methylenetetrahydrofolate + dUMP =
dihydrofolate + dTMP.
[PATHWAY] DEOXYRIBONUCLEOTIDE BIOSYNTHESIS.
[SUBUNIT] HOMODIMER.
[SIMILARITY] BELONGS TO THE THYMIDYLATE SYNTHASE FAMILY.
FEATURES Location/Qualifiers
source 1..313
/organism="Homo sapiens"
/db_xref="taxon:9606"
gene 1..313
/gene="TYMS"
/note="TS"
Protein 1..313
/gene="TYMS"
/product="Thymidylate synthase"
/EC_number="2.1.1.45"
Site 195
/gene="TYMS"
/site_type="active"
ORIGIN
1 mpvagselpr rplppaaqer daeprpphge lqylgqiqhi lrcgvrkddr tgtgtlsvfg
61 mqaryslrde fpllttkrvf wkgvleellw fikgstnake lsskgvkiwd angsrdflds
121 lgfstreegd lgpvygfqwr hfgaeyrdme sdysgqgvdq lqrvidtikt npddrriimc
181 awnprdlplm alppchalcq fyvvnselsc qlyqrsgdmg lgvpfniasy alltymiahi
241 tglkpgdfih tlgdahiyln hieplkiqlq reprpfpklr ilrkvekidd fkaedfqieg
301 ynphptikme mav
//

Revised: October 24, 2001.

□1: XP_046765. thymidylate synth...[gi:14762323]
BLink, Nucleotide, Related Sequences, Taxonomy,
LinkOut

LOCUS       XP_046765                313 aa            linear   PRI 07-FEB-2002
DEFINITION  thymidylate synthetase [Homo sapiens].
ACCESSION   XP_046765
PID         g14762323
VERSION     XP_046765.1  GI:14762323
DBSOURCE    REFSEQ: accession XM_046765.1
KEYWORDS    .
SOURCE      human.
  ORGANISM  Homo sapiens
            Eukaryota; Metazoa; Chordata; Craniata; Vertebrata; Euteleostomi;
            Mammalia; Eutheria; Primates; Catarrhini; Hominidae; Homo.
REFERENCE   1  (residues 1 to 313)
  AUTHORS   NCBI Annotation Project.
  TITLE     Direct Submission
  JOURNAL   Submitted (06-FEB-2002) National Center for Biotechnology
            Information, NIH, Bethesda, MD 20894, USA
COMMENT     GENOME ANNOTATION REFSEQ:  This model reference sequence was
            predicted from NCBI contig NT_011005 by automated computational
            analysis using gene prediction method: BLAST, supported by mRNA
            evidence.
            Also see:
                Documentation of NCBI's Annotation Process

FEATURES             Location/Qualifiers
     source          1..313
                     /organism="Homo sapiens"
                     /db_xref="taxon:9606"
                     /chromosome="18"
     Protein         1..313
                     /product="thymidylate synthetase"
     Region          28..313
                     /region_name="Thymidylate synthase"
                     /note="thymidylat_synt"
                     /db_xref="CDD:pfam00303"
     CDS             1..313
                     /gene="TYMS"
                     /coded_by="XM_046765.1:70..1011"
                     /note="Located on Accession NT_011005"
ORIGIN
        1 mpvagselpr rplppaaqer daeprpphge lqylgqiqhi lrcgvrkddr tgtgtlsvfg
       61 mqaryslrde fpllttkrvf wkgvleellw fikgstnake lsskgvkiwd angsrdflds
      121 lgfstreegd lgpvygfqwr hfgaeyrdme sdysgqgvdq lqrvidtikt npddrriimc
      181 awnprdlplm alppchalcq fyvvnselsc qlyqrsgdmg lgvpfniasy alltymiahi
      241 tglkpgdfih tlgdahiyln hieplkiqlq reprpfpklr ilrkvekidd fkaedfqieg
      301 ynphptikme mav
//

Revised: October 24, 2001.

Disclaimer | Write to the Help Desk
NCBI | NLM | NIH

## Claims

1. Thymidylate synthase mutants, differing from human wildtype thymidylate synthase in single mutations at positions:
E23, T53, V84, K93, D110, D116, M190, P194, S206, M219, H250, D254, Y258 or K284,
where further silent or non-functional mutations with respect to TS activity are not excluded.

2. Thymidylate synthase mutants as claimed in claim 1 wherein said single mutations are E23G, T53S, V84A, K93E, D110E, D116A, M190L, P194Q, S206G, M219V, H250L, D254E, D254A, D254N, Y258S, Y258F or K284N.

3. Thymidylate synthase mutants differing from human wildtype thymidylate synthase in double or multiple mutations at positions:
55, 106, and 284;
5, 78, and 219;
45 and 254;
193 and 231;
6, 69, and 211;
5, 13, and 231;
103 and 204;
142 and 225;
17, 116, and 254;
117, 169, and 254;
120 and 278;
38 and 104;
8, 81, 131, and 230;
51, 82, 99, and 171;
167 and 192,
where further silent or non-functional mutations with respect to TS activity are not excluded.

4. Thymidylate synthase mutants as claimed in claim 3 wherein said double or multiple mutations are
T55I, V106A, and K284I;
G5S, R78C, and M219I;
V45A and D254N;
P193S and A231G;
S6N, D69Q, and Q211L;
G5D, L13R,and A231T;
S103T and V204A;
F142S and F225I;
A17T, D116A, and D254E;
F117S, K169R, and D254E;
S120T and K278R;
Q38H and K104D;
L8Q, W81G, L131V, and Y230F;
T51S, K82Q, K99D, and N171S;
T167S and L192P,

5. Rekombinant cDNA, encoding a TS mutant according to one of claims 1 to 4.

6. DNA vector comprising one or more cDNA copies of claim 5 and optionally other components.

7. Fusion protein comprising a transductor for the transduction of a protein into a human cell and a human thymidylate synthase mutant according to one of claims 1 to 4 coupled to said transductor.

8. Host cell transfected with a cDNA according to claim 5 or a DNA vector according to claim 6 or comprising TS mutants according to one of claims 1 to 4, and expressing at least one of the thymidylate synthase mutants according to claims 1 to 4.

9. Pharmaceutical composition for treatment and protection of human cell populations against the toxic manifestation of analogs that inhibit thymidylate synthase comprising one or more TS mutants according to one of claims 1 to 4, one or more cDNAs of claim 5, vectors of claim 6, or fusion proteins of claim 7.

10. The use of protein mutants, respective DNAs, vectors or fusion proteins according to one of claims 1 to 7 for transfection of human cells which may be affected by side effects due to chemotherapy with thymidylate synthase inhibitors, for the *ex-vivo* or *in vivo* transfection of bone marrow cells, or for transfection of early progenitor cells separated or grown from bone marrow cells.

11. The use of protein mutants, respective DNAs, vectors or fusion proteins according to one of claims 1 to 7 for the identification of new nucleotide analogs that inhibit normal human thymidylate synthase.

12. The use of a cDNA according to claim 5 or vectors according to claim 6 with gene therapeutical metods.

13. The use of protein mutants, respective DNAs, vectors or fusion proteins according to one of claims 1 to 7 for protection of human cell populations, preferably mucosa, against the toxic manifestation of analogs that inhibit TS.

14. The use of protein mutants, respective DNAs, vectors or fusion proteins according to one of claims 1 to 7 for the manufacture of pharmaceutical and gene therapeutic compositions for the protection of human mucosa against ulceration under chemotherapy with TS inhibitors. ML
